# EUROPEAN PATENT APPLICATION

(11) **EP 2 314 707 A1**
(43) Date of publication of application: **27.04.2011**
(21) Application number: 09174068.8
(22) Date of filing: 26.10.2009
(51) Int. Cl.: C12N 15/85, C12N 5/10

(54) **Method for the expression of a recombinant protein in a mammalian cell**

(71) Applicant: Amarna Therapeutics B.V., 2333 AL Leiden (NL)
(72) Inventor: De Vries, Walter Gerhardus, 2333 AL Leiden (NL)
(74) Representative: Habets, Winand

(57) **Abstract**

The invention relates to methods for the production of a recombinant protein in a mammalian cell and methods to enhance the production of recombinant proteins in mammalian cells. More in particular, the invention provides a cell for the production of a recombinant protein of interest wherein said cell is permissive to a polyomavirus and wherein said cell comprises the genetic elements A and B wherein A encodes a polyomaviral large T antigen or a functional equivalent thereof and B comprises a gene encoding a protein of interest under the functional control of a polyomaviral origin of replication or a functional equivalent thereof, wherein said cell lacks the capability to express a polyomaviral small T antigen or a functional equivalent thereof as well as the capability to express a polyomavirus capsid protein.

SEQUENCE LISTING

<110>  Amarna Therapeutics B.V.

<120>  Method for the expression of a recombinant protein in a mammalian
        cell

<130>  123 EPP0

<160>  33

<170>  PatentIn version 3.5

<210>  1
<211>  51
<212>  DNA
<213>  Artificial Sequence
<220>
<223>  Probes and Primers

<400>  1
gccgctttat taattaagcc ctgcaggttg tttaaacttg gcgcgcctta t                51

<210>  2
<211>  50
<212>  DNA
<213>  Artificial Sequence
<220>
<223>  Probes and Primers

<400>  2
cgaaataatt aattcgggac gtccaacaaa tttgaaccgc gcggaatagc                  50

<210>  3
<211>  14
<212>  DNA
<213>  Artificial Sequence
<220>
<223>  Probes and Primers

<400>  3
cggcggccgc gtac                                                         14

<210>  4

<211>  43
<212>  DNA
<213>  Artificial Sequence
<220>
<223>  Probes and Primers

<400>  4
ttggcgcgcc tcaatattgg ccattagcca tattattcat tgg                          43

<210>  5
<211>  44
<212>  DNA
<213>  Artificial Sequence
<220>
<223>  Probes and Primers

<400>  5
gctaggtcgg aggcgccggc ccttgccacg taaccttcga acag                         44

<210>  6
<211>  38
<212>  DNA
<213>  Artificial Sequence
<220>
<223>  Probes and Primers

<400>  6
ccacccaagc ttatgaccga gtacaagccc acggtgcg                                38

<210>  7
<211>  38
<212>  DNA
<213>  Artificial Sequence
<220>
<223>  Probes and Primers

<400>  7
cgtactgggc gttcgggcca cggactgagc tcgcctat                                38

<210>  8

<211>  37
<212>  DNA
<213>  Artificial Sequence
<220>
<223>  Probes and Primers

<400>  8
caaccgctcg agctgtgcct tctagttgcc agccatc                                37

<210>  9
<211>  31
<212>  DNA
<213>  Artificial Sequence
<220>
<223>  Probes and Primers

<400>  9
cggggtaccc catagagccc accgcatccc c                                      31

<210>  10
<211>  40
<212>  DNA
<213>  Artificial Sequence
<220>
<223>  Probes and Primers

<400>  10
accatggata aagttttaaa cagagaggaa tctttgcagc                             40

<210>  11
<211>  33
<212>  DNA
<213>  Artificial Sequence
<220>
<223>  Probes and Primers

<400>  11
ttatgtttca ggttcagggg gaggtgtggg agg                                    33

<210>  12

<211>  53
<212>  DNA
<213>  Artificial Sequence
<220>
<223>  Probes and Primers

<400>  12
tcctgcaggc ggggtaccct agtctagact agccgcgggg agtttaaaca gct          53

<210>  13
<211>  53
<212>  DNA
<213>  Artificial Sequence
<220>
<223>  Probes and Primers

<400>  13
gtttaaactc cccgcggcta gtctagacta gggtaccccg cctgcaggag tac          53

<210>  14
<211>  34
<212>  DNA
<213>  Artificial Sequence
<220>
<223>  Probes and Primers

<400>  14
gcaggctacc atggataaag ttttaaacag agag                               34

<210>  15
<211>  55
<212>  DNA
<213>  Artificial Sequence
<220>
<223>  Probes and Primers

<400>  15
gaaacctccg aagaccctac gttgactcta aggttggata ccttgactac ttacc        55

<210>  16

<211>  56
<212>  DNA
<213>  Artificial Sequence
<220>
<223>  Probes and Primers

<400>  16
ctttggaggc ttctgggatg caactgagat tccaacctat ggaactgatg aatggg          56

<210>  17
<211>  33
<212>  DNA
<213>  Artificial Sequence
<220>
<223>  Probes and Primers

<400>  17
aggaatgttg tacaccatgc attttaaaaa gtc                                   33

<210>  18
<211>  43
<212>  DNA
<213>  Artificial Sequence
<220>
<223>  Probes and Primers

<400>  18
ccgctcgagt tgcggccgct gtgccttcta gttgccagcc atc                        43

<210>  19
<211>  34
<212>  DNA
<213>  Artificial Sequence
<220>
<223>  Probes and Primers

<400>  19
ggtaccatag agcccaccgc atccccagca tgcc                                  34

<210>  20

<211>  52
<212>  DNA
<213>  Artificial Sequence
<220>
<223>  Probes and Primers

<400>  20
ggccgcttta ttaattaagc cctgcaggtt gtttaaactt ggcgcgcctt at          52

<210>  21
<211>  50
<212>  DNA
<213>  Artificial Sequence
<220>
<223>  Probes and Primers

<400>  21
cgataaggcg cgccaagttt aaacaacctg cagggcttaa ttaataaagc          50

<210>  22
<211>  28
<212>  DNA
<213>  Artificial Sequence
<220>
<223>  Probes and Primers

<400>  22
cgggatccag acatgataag atacattg          28

<210>  23
<211>  42
<212>  DNA
<213>  Artificial Sequence
<220>
<223>  Probes and Primers

<400>  23
atagtttagc ggccgcaact tgtttattgc agcttataat gg          42

<210>  24

<211>  35
<212>  DNA
<213>  Artificial Sequence
<220>
<223>  Probes and Primers

<400>  24
tctaggcgcg ccgatggata aagttttaaa cagag                                35

<210>  25
<211>  55
<212>  DNA
<213>  Artificial Sequence
<220>
<223>  Probes and Primers

<400>  25
gaaacctccg aagaccctac gttgactcta aggttggata ccttgactac ttacc          55

<210>  26
<211>  56
<212>  DNA
<213>  Artificial Sequence
<220>
<223>  Probes and Primers

<400>  26
ctttggaggc ttctgggatg caactgagat tccaacctat ggaactgatg aatggg         56

<210>  27
<211>  29
<212>  DNA
<213>  Artificial Sequence
<220>
<223>  Probes and Primers

<400>  27
tccttaatta attatgtttc aggttcagg                                       29

<210>  28

<211>  46
<212>  DNA
<213>  Artificial Sequence
<220>
<223>  Probes and Primers

<400>  28
gcagtactgg tttaaaccag atctggcgcc cctgcagggg atccta                    46

<210>  29
<211>  46
<212>  DNA
<213>  Artificial Sequence
<220>
<223>  Probes and Primers

<400>  29
taggatcccc tgcaggggcg ccagatctgg tttaaaccag tactgc                    46

<210>  30
<211>  41
<212>  DNA
<213>  Artificial Sequence
<220>
<223>  Probes and Primers

<400>  30
agctttagtt taaacacaag tttgtacaaa aaagctgaac g                         41

<210>  31
<211>  34
<212>  DNA
<213>  Artificial Sequence
<220>
<223>  Probes and Primers

<400>  31
agataccctg caggaccact ttgtacaaga aagc                                 34

<210>  32

<211>  48
<212>  DNA
<213>  Artificial Sequence
<220>
<223>  Probes and Primers

<400>  32
gggacaagtt tgtacaaaaa agcaggctta atgctgctgc tgctgctg                    48

<210>  33
<211>  48
<212>  DNA
<213>  Artificial Sequence
<220>
<223>  Probes and Primers

<400>  33
ggggaccact ttgtacaaga aagctgggta tcatgtctgc tcgaagcg                    48

SEQUENCE LISTING

<110>  Amarna Therapeutics B.V.

<120>  Method for the expression of a recombinant protein in a mammalian
        cell

<130>  123 EPPO

<160>  33

<170>  PatentIn version 3.5

<210>  1
<211>  51
<212>  DNA
<213>  Artificial Sequence

<220>
<223>  Probes and Primers

<400>  1
gccgctttat taattaagcc ctgcaggttg tttaaacttg gcgcgcctta t                51

<210>  2
<211>  50
<212>  DNA
<213>  Artificial Sequence

<220>
<223>  Probes and Primers

<400>  2
cgaaataatt aattcgggac gtccaacaaa tttgaaccgc gcggaatagc                50

<210>  3
<211>  14
<212>  DNA
<213>  Artificial Sequence

<220>
<223>  Probes and Primers

<400>  3
cggcggccgc gtac                                                        14

<210>  4
<211>  43
<212>  DNA
<213>  Artificial Sequence

<220>
<223>  Probes and Primers

<400>  4
ttggcgcgcc tcaatattgg ccattagcca tattattcat tgg                        43

<210>  5
<211>  44
<212>  DNA
<213>  Artificial Sequence

<220>
<223>  Probes and Primers

<400>  5
gctaggtcgg aggcgccggc ccttgccacg taaccttcga acag                        44


<210>  6
<211>  38
<212>  DNA
<213>  Artificial Sequence

<220>
<223>  Probes and Primers

<400>  6
ccacccaagc ttatgaccga gtacaagccc acggtgcg                               38


<210>  7
<211>  38
<212>  DNA
<213>  Artificial Sequence

<220>
<223>  Probes and Primers

<400>  7
cgtactgggc gttcgggcca cggactgagc tcgcctat                               38


<210>  8
<211>  37
<212>  DNA
<213>  Artificial Sequence

<220>
<223>  Probes and Primers

<400>  8
caaccgctcg agctgtgcct tctagttgcc agccatc                                37


<210>  9
<211>  31
<212>  DNA
<213>  Artificial Sequence

<220>
<223>  Probes and Primers

<400>  9
cggggtaccc catagagccc accgcatccc c                                      31


<210>  10
<211>  40
<212>  DNA
<213>  Artificial Sequence

<220>
<223>  Probes and Primers

<400>  10
accatggata aagttttaaa cagagaggaa tctttgcagc                             40


<210>  11
<211>  33
<212>  DNA
<213>  Artificial Sequence

<220>
<223>  Probes and Primers

<400>  11
ttatgtttca ggttcagggg gaggtgtggg agg                                    33


<210>  12
<211>  53
<212>  DNA
<213>  Artificial Sequence

<220>
<223>  Probes and Primers

<400>  12
tcctgcaggc ggggtaccct agtctagact agccgcgggg agtttaaaca gct              53


<210>  13
<211>  53
<212>  DNA
<213>  Artificial Sequence

<220>
<223>  Probes and Primers

<400>  13
gtttaaactc cccgcggcta gtctagacta gggtaccccg cctgcaggag tac              53


<210>  14
<211>  34
<212>  DNA
<213>  Artificial Sequence

<220>
<223>  Probes and Primers

<400>  14
gcaggctacc atggataaag ttttaaacag agag                                   34


<210>  15
<211>  55
<212>  DNA
<213>  Artificial Sequence

<220>
<223>  Probes and Primers

<400>  15
gaaacctccg aagaccctac gttgactcta aggttggata ccttgactac ttacc            55


<210>  16
<211>  56
<212>  DNA
<213>  Artificial Sequence

<220>
<223>  Probes and Primers

<400>  16
ctttggaggc ttctgggatg caactgagat tccaacctat ggaactgatg aatggg           56


<210>  17
<211>  33

<212>  DNA
<213>  Artificial Sequence

<220>
<223>  Probes and Primers

<400>  17
aggaatgttg tacaccatgc attttaaaaa gtc                                    33


<210>  18
<211>  43
<212>  DNA
<213>  Artificial Sequence

<220>
<223>  Probes and Primers

<400>  18
ccgctcgagt tgcggccgct gtgccttcta gttgccagcc atc                         43


<210>  19
<211>  34
<212>  DNA
<213>  Artificial Sequence

<220>
<223>  Probes and Primers

<400>  19
ggtaccatag agcccaccgc atccccagca tgcc                                   34


<210>  20
<211>  52
<212>  DNA
<213>  Artificial Sequence

<220>
<223>  Probes and Primers

<400>  20
ggccgcttta ttaattaagc cctgcaggtt gtttaaactt ggcgcgcctt at               52


<210>  21
<211>  50
<212>  DNA
<213>  Artificial Sequence

<220>
<223>  Probes and Primers

<400>  21
cgataaggcg cgccaagttt aaacaacctg cagggcttaa ttaataaagc                  50


<210>  22
<211>  28
<212>  DNA
<213>  Artificial Sequence

<220>
<223>  Probes and Primers

<400>  22
cgggatccag acatgataag atacattg                                          28

<210>  23
<211>  42
<212>  DNA
<213>  Artificial Sequence

<220>
<223>  Probes and Primers

<400>  23
atagtttagc ggccgcaact tgtttattgc agcttataat gg                          42


<210>  24
<211>  35
<212>  DNA
<213>  Artificial Sequence

<220>
<223>  Probes and Primers

<400>  24
tctaggcgcg ccgatggata aagttttaaa cagag                                  35


<210>  25
<211>  55
<212>  DNA
<213>  Artificial Sequence

<220>
<223>  Probes and Primers

<400>  25
gaaacctccg aagaccctac gttgactcta aggttggata ccttgactac ttacc            55


<210>  26
<211>  56
<212>  DNA
<213>  Artificial Sequence

<220>
<223>  Probes and Primers

<400>  26
ctttggaggc ttctgggatg caactgagat tccaacctat ggaactgatg aatggg           56


<210>  27
<211>  29
<212>  DNA
<213>  Artificial Sequence

<220>
<223>  Probes and Primers

<400>  27
tccttaatta attatgtttc aggttcagg                                         29


<210>  28
<211>  46
<212>  DNA
<213>  Artificial Sequence

<220>
<223>  Probes and Primers

<400>  28
gcagtactgg tttaaaccag atctggcgcc cctgcagggg atccta                      46


<210>  29
<211>  46
<212>  DNA
<213>  Artificial Sequence

<220>
<223>  Probes and Primers

<400>  29
taggatcccc tgcaggggcg ccagatctgg tttaaaccag tactgc                      46


<210>  30
<211>  41
<212>  DNA
<213>  Artificial Sequence

<220>
<223>  Probes and Primers

<400>  30
agctttagtt taaacacaag tttgtacaaa aaagctgaac g                           41


<210>  31
<211>  34
<212>  DNA
<213>  Artificial Sequence

<220>
<223>  Probes and Primers

<400>  31
agataccctg caggaccact ttgtacaaga aagc                                   34


<210>  32
<211>  48
<212>  DNA
<213>  Artificial Sequence

<220>
<223>  Probes and Primers

<400>  32
gggacaagtt tgtacaaaaa agcaggctta atgctgctgc tgctgctg                    48


<210>  33
<211>  48
<212>  DNA
<213>  Artificial Sequence

<220>
<223>  Probes and Primers

<400>  33
ggggaccact ttgtacaaga aagctgggta tcatgtctgc tcgaagcg                    48

## Description

### Field of the invention

The invention relates to methods for the production of a recombinant protein in a mammalian cell and methods to enhance the production of recombinant proteins in mammalian cells.

### Background of the invention

The use of mammalian expression systems for producing therapeutic recombinant proteins such as antibodies, growth factors and hormones, viruses or viral vectors has been well documented. Mammalian cells have the ability to carry out authentic protein folding and complex post-translational modifications, which are necessary for the therapeutic activity of many proteins. As such, a number of mammalian cell lines have been approved by regulatory bodies for use in the production of therapeutic proteins, viruses or viral vectors.

Chinese Hamster ovary (CHO) cell lines are routinely used for the production of therapeutic proteins. A number of characteristics make CHO cells very suitable as producer cells: high protein levels can be reached in CHO cells; they provide a safe production system free of infectious or virus-like particles; they have been characterized extensively; they can grow in suspension to high cell densities in bioreactors, using serum-free culture media. The cell line CHO-K1 has formed the basis for the generation of a variety of CHO cell line derivatives with improved characteristics, such as the Super-CHO cell line (Pak S.C.O. et al., Cytotechnology 22: 139-146, 1996). Super-CHO cells were derived from CHO-K1 cells, which were genetically engineered to express the genes encoding transferrin and the insulin-like growth factor, IGF-1.

African Green Monkey kidney cells (Vero) are certified for the production of rabies, polio and influenza virus particles for use as vaccines. The cell line is recommended by the World Health Organisation for vaccine production for human use (World Health Organisation. WHO Technical Report Series vol. 878, WHO Geneva, pp. 20-53, annex 1, 1998). A number of characteristics make Vero cells very suitable as producer cells: The cell line has a defect in the antiviral interferon pathway and as a result is highly permissive for the majority of human viruses and accumulating virus particles in high amounts; it provides a safe production system free of infectious or virus-like particles; it has been characterized extensively and the cells can grow in suspension to high cell densities in bioreactors using serum-free culture media.

Recombinant therapeutic proteins are generally produced in mammalian cells by transfecting said cells with DNA molecules encoding the therapeutic protein(s) and a selectable marker. A cell clone that stably produces the therapeutic protein(s) from gene copies that are integrated into the chromosomal DNA is subsequently selected using the selectable marker. The selection of such a cell clone is a costly and time-consuming process. The yields of therapeutic proteins produced in mammalian cells using said method are in general low compared to the yields of proteins produced in prokaryote cells, despite the use of strong promoters and/ or multicopy transgene insertions or of other ways to enhance the transcription. Overall, recombinant therapeutic proteins produced in mammalian cells are expensive and there is a need to reduce the costs of the production of said proteins by optimising the production methods and/or by developing alternative gene expression systems that provide increased yields of therapeutic proteins in mammalian cells.

Viral replication competent vectors or replicons have been used for a long time as an alternative expression system to increase the yields of therapeutic proteins in mammalian cells. The target gene(s) can be expressed under transcriptional control of viral promoters whereby the mRNAs accumulate to extremely high levels in the cytoplasm after transfection and upon replication, yielding large amounts of target protein.

Replicon-based expression systems based on RNA viruses such as alphaviruses in general produce recombinant proteins for only a short period of time after transfection. This, in combination with the high mutation rate of replicating RNA compared to replicating DNA makes RNA virus-derived replicons unattractive for commercial application.

Because of their small circular DNA genomes and episomal replication property polyomavirus-based replicons are of great interest as expression system in mammalian cells to enhance the production of therapeutic proteins.

Polyomaviruses are comprised of a family of non-enveloped DNA viruses with icosahedral capsids. They are isolated from a variety of animal species including humans, monkeys, rodents and birds. Three rodent polyomaviruses have been identified: murine polyomavirus (MuPyV), murine pneumotropic virus (MptV) and hamster polyomavirus (HaPyV). Many primate polyomaviruses have been described of which SV40 is the most well-known. SV40 has a 5.25 kilo base pair, long circular double stranded DNA genome. The SV40 genome consists of two regulatory regions, the origin of replication region and the polyadenylation region. The origin of replication region is 500 base pairs long and comprises two oppositely-directed promoters, the early and late promoter (SVEP and SVLP respectively), the origin of replication and the packaging signal. The polyadenylation region is 100 base pairs long and contains the polyadenylation signals of both the early and the late transcripts. SVEP drives expression of the early primary transcript that is spliced by host-encoded splicing factors into 2 different mRNAs encoding small and large tumor (T) antigens (STag and LTag, respectively). In some polyomaviruses including the rodent polyomaviruses the early primary transcript is spliced into 3 different mRNAs encoding small, middle and large T antigens (Stag, MTag and LTag, respectively). SVLP drives expression of the late primary transcript that is spliced by host-encoded splicing factors into different mRNAs encoding the viral capsid proteins VP1, 2 and 3.

It is well documented in the prior art that all T antigens are required for efficient virus replication. The SV40 T antigens cooperatively immortalize primary mammalian cells, transform established mammalian cell lines and induce tumours in immuno-compromized young-borne rodents (Brady J., et al., Proceedings of the National Academy of Sciences USA 81: 2040-2044, 1984). A number of reports suggest that SV40 infections are associated with human malignancies, caused by the oncogenic activity of the chronically expressed T antigens (Butel J.S. and Lednicky J.A. Journal of the National Cancer Institute 91: 119-134, 1999).

Large T antigen accumulates in the nucleus of infected cells and is the replicase-associated protein required for episomal DNA replication and for activation of the SVLP.

Small T antigen accumulates in the cytoplasm of infected cells. The precise role of the small T antigen in virus replication has remained unclear. Infection of SV40-permissive cells with SV40 mutants that do not encode the small T antigen such as dl883 leads to reduced growth rate and virus yields compared to those infected with wildtype SV40 (Sugano S., et al., Journal of Virology 41: 1073-1075, 1982). In one study it has been found that the absence of the coding capacity for the small T antigen in said SV40 mutants has an adverse effect on the virus yields in infected cells, because a significant portion of the cells infected with said mutants does not divide and as a consequence does not start to produce viral DNA. From this study it was concluded that the small T antigen assists the large T antigen in replicating viral DNA in SV40-permissive cells (Gauchat J-F. and Weil R., Nucleic Acids Research 14: 9339-9351, 1986). A study of Bikel and Loeken using a series of small T antigen SV40 mutants demonstrated that the small T antigen has an additive effect on large T antigen-mediated activation of the SVLP. From this study it was concluded that the small T antigen assists the large T antigen in activating the SVLP resulting in an increased number of virus particles in SV40-permissive cells (Bikel I. and Loeken M.R., Journal of Virology 66: 1489-1494, 1992).

The role of the middle T antigen in polyomavirus replication has remained unclear.

Polyomaviral replicons can be divided into three categories: early replacement replicons harbouring the polyomaviral origin of replication and the capsid protein coding region, early plus late replacement replicons harbouring the origin of replication, and late replacement replicons harbouring the origin of replication and the T antigen coding region (Hammarskjöld M-L., in: Methods in Molecular Biology, Edited by Murray E.J., volume 7: 169-180, 1991).

Early replacement polyomaviral replicons and early plus late polyomaviral replicons are replication-incompetent in mammalian cells lacking the polyomaviral T antigens. Said replicons exclusively replicate in cells permissive to the cognate polyomavirus that accumulate the polyomaviral T antigens. Examples of such cells are the simian COS cell lines derived from monkey CV1 cells, Verots cell lines derived from Vero, CHOP cell lines derived from CHO-K1 and HEK293T or HEK293TT cell lines derived from HEK293.

COS cell lines such as COS-1 and COS-7 were generated by transformation of monkey CV1 cells with SV40 DNA (Gluzman Y., Cell 23: 175 - 182, 1981). In COS cells the replication of SV40-derived early and early plus late replacement replicons overwhelms and kills the host cell within a few days after transfection, which makes this expression system not attractive for commercial application (Aruffo A., Current Protocols in Neuroscience 4.7.1-4.7.7, 1998). The Verots cell lines were generated by transformation of Vero cells with origin of replication defective SV40 DNA encoding a wildtype small T antigen and a temperature sensitive large T antigen (Ohno T. et al., Cytotechnology 7: 165-172, 1991). Verots S3 supported the replication of an early plus late replacement SV40 replicon encoding the human Growth Hormone (hGH) leading to the production of large amounts of hGH at 33 Degrees Celsius, whereas at 37 Degrees Celsius the production of hGH lasts for only a short period of time after transfection.

The CHOP cell lines were generated by introducing the mouse polyomavirus early region into the chromosomal DNA of CHO-K1 cells (Heffernan M. and Dennis J.W., Nucleic Acids Research 19: 85-92, 1991). A number of CHOP cell lines supported replication of replicon plasmid early plus late replacement replicon CDM8 (invitrogen), a mammalian replicon plasmid carrying the murine polyomavirus origin of replication. The replicon DNA is lost within 3 days after transfection due to degradation and/or cell division and the expression of the desired protein was shown to only last 48-72 hours, not enough to make this system attractive for commercial application. It was reported that the addition of a gene cassette encoding the Epstein-Barr Virus (EBV) nuclear antigen-1 (EBNA-1) and OriP to an early plus late replacement polyomaviral replicon encoding hGH resulted in prolonged expression of hGH (Kunaparaju R. et al., Biotechnology and Bioengineering 91: 670-677, 2005).

A derivative of the HEK293 cell line is the HEK293T cell line, expressing the SV40 early region under transcriptional control of the Rous Sarcoma virus long terminal repeat promoter. Vera et al. found that HEK293T poorly supports the replication of early replacement SV40 replicons (Vera M., et al., Molecular Therapy 10: 780-791, 2004). The HEK293TT cell line has been developed as a derivative of HEK293T, generated by stable transfection with a gene construct encoding the SV40 large T antigen. HEK293TT cells are used for the production of recombinant human papilloma virus (HPV) pseudo-vector particles. The recombinant HPV pseudo-vector particles are produced in HEK293TT by transfecting the cells with early plus late replacement SV40 replicon DNA that harbours the HPV capsid genes and DNA of a replicon that harbours an HPV pseudo-genome (Buck C.B. et al., Methods in Molecular Medicine 119: 445-462, 2005). Since both HEK293T and HEK293TT accumulate the T antigen oncogenes and poorly support the replication of early and early plus late replacement SV40 replicons, the use of these cell lines to produce therapeutic proteins is also undesired and impractical.

Late replacement polyomaviral replicons harbour the polyomaviral origin of replication and encode the polyomaviral T antigens and as a result are replication-competent in mammalian cells permissive to the cognate polyomavirus. Since expression of the viral capsid proteins from the late promoter is induced by the polyomaviral T antigens, the late promoter in the late replacement polyomaviral replicons has a strong promoter activity in cells permissive to the polyomavirus compared to other promoters used in the art such as the human cytomegalovirus immediate early promoter or the SVEP. The major advantages of the use of late replacement polyomaviral replicons for the production of therapeutic proteins in mammalian cells is the fact that said mammalian cells do not need to be genetically modified and that the therapeutic proteins can be expressed from the strong late polyomaviral promoter. Expression of influenza A virus haemagglutinin variants in monkey CV1 cells using a late replacement SV40 vector resulted in high yields of these glycosylated membrane-bound proteins although the expression of haemagglutinin again lasted for a short period of time (Naim H.Y. and Roth M.G., Journal of Virology 67: 4831-4841, 1993). A study by La Bella and Ozer demonstrated that a late replacement replicon based on murine polyomavirus replicates in CHO cells (La Bella F. and Ozer H.L., Virus Research 2: 329-343, 1985). The disadvantages of late replacement polyomaviral replicon expression systems to date is that the mammalian cells harbour DNA encoding the polyomaviral T antigen oncoproteins and that the expression of the desired protein was shown to only last for a short period of time after introduction of the replicon DNA into the mammalian cells. These disadvantages make late replacement polyomaviral replicons unattractive for commercial application.

It has been demonstrated that the mammalian innate intracellular immune system senses viral infection by recognizing viral nucleic acid signatures in the cytoplasm of infected cells and activates potent antiviral responses. Besides the interferon (IFN) pathway (that is absent in Vero cells), there is accumulating evidence that RNA silencing or RNA interference (RNAi) serves as a cytoplasmic antiviral mechanism in mammalian cells (De Vries W. et al., Gene Therapy 15: 545-552, 2008). Mammalian viruses encode proteins that inhibit RNAi in the cytoplasm of infected cells and therefore serve as RNA silencing suppressors (De Vries W. and Berkhout B. International Journal of Biochemistry and Cell Biology 40: 2007-2012, 2008).

Patent application WO 04/035796 describes a number of RNAi suppressors encoded by vertebrate viruses and teaches that the introduction of said proteins in a mammalian cell results in increased transgene expression and virus replication. Expression of said viral RNAi suppressor proteins in the cytoplasm of mammalian cells is detrimental to the cells. The use of viral RNAi suppressor proteins to improve polyomaviral replicon expression systems is therefore impractical.

There thus remains a need for an improved mammalian gene expression system, which can be used for the safe and efficient production of recombinant proteins, in particular therapeutic proteins.

It is an object of the present invention to overcome or ameliorate at least one of the disadvantages of the prior art, or to provide a useful alternative.

### Summary of the invention

The above objects have been met by the present invention in that a mammalian cell is provided for the production of a recombinant protein of interest wherein said cell is permissive to a polyomavirus and wherein said cell comprises the genetic elements A and B wherein A encodes a polyomaviral large T antigen or a functional equivalent thereof and B comprises a gene encoding a protein of interest under the functional control of a polyomaviral origin of replication or a functional equivalent thereof, wherein said cell lacks the capability to express a polyomaviral small T antigen or a functional equivalent thereof as well as the capability to express a polyomavirus capsid protein.

Also provided by the present invention is a method for the production of a recombinant protein of interest in a mammalian cell permissive to a polyomavirus comprising the genetic elements A and B wherein A encodes a polyomaviral large T antigen or a functional equivalent thereof and B comprises a gene encoding a protein of interest under the functional control of a polyomaviral origin of replication or a functional equivalent thereof, wherein said cell lacks the capability to express a polyomaviral small T antigen or a functional equivalent thereof as well as the capability to express a polyomavirus capsid protein, the method further comprising the step of culturing said cell under conditions allowing expression of the recombinant protein of interest and harvesting the recombinant protein of interest from the cell culture.

Cells and cell lines for use in this invention may be derived from conventional mammalian cell lines permissive for a polyomavirus, such as Vero or CHO cell lines.

### Detailed description of the invention

The present inventors found that the polyomaviral small T antigen has RNAi suppressor activity capable of transactivating reporter gene activity and interfering with micro-RNA (miRNA) activity in mammalian cells. They further found that accumulation of small T antigen in the cytoplasm of mammalian cells, just as that of other viral RNAi suppressors, is detrimental to the cells particularly when the small T antigen protein is expressed at a high level from a replicating DNA molecule e.g. a late replacement polyomaviral replicon.

It has been well documented that late replacement polyomaviral replicons overwhelm and kill cells permissive to the cognate polyomavirus, and as a result the expression of recombinant protein(s) using said replicons only lasts for a short period of time (a few days) after introduction of replicon DNA into the cells (Aruffo A., Current Protocols in Neuroscience 4.7.1-4.7.7, 1998). Such systems are not attractive for the production of recombinant proteins since the total amount of recombinant protein of interest produced is generally low.

Polyomaviral replicons lacking the small T antigen have been described (Gauchat et al., Nucl. Acids Res. 14, 9339-9351, 1988). In cells permissive to SV40 and harbouring said polyomaviral replicons, the synthesis of SV40 large T antigen in the absence of small T antigen was found sufficient to induce mitosis in 50 - 60% of the cells and to subsequently initiate replication of said replicons. Gauchat et al. conclude that the synthesis of small T antigen is required for the production of viral progeny DNA in the remaining 40-50% of cells and to initiate replication of said replicons in all cells. Hence, these cells are unsuited for the efficient production of recombinant protein.

The present inventors now found that efficient production of a recombinant protein of interest may be achieved employing a polyomaviral expression system that lacks the small T antigen as well as a viral capsid protein.

The present invention offers a solution to the short-term expression problem relating to the use of late replacement polyomaviral replicon expression systems, making said systems attractive for commercial application.

According to a first aspect, the present invention provides a mammalian cell for the production of a recombinant protein of interest wherein said cell is permissive to a polyomavirus and wherein said cell comprises the genetic elements A and B wherein A encodes a polyomaviral large T antigen or a functional equivalent thereof and B is a gene of interest under the functional control of the polyomaviral origin of replication or a functional equivalent thereof, wherein said cell lacks the capability to express a polyomaviral small T antigen or a functional equivalent thereof as well as the capability to express a polyomavirus capsid protein.

Also provided by the present invention is a method for the production of a recombinant protein of interest in a mammalian cell permissive to a polyomavirus comprising the genetic elements A and B wherein A encodes a polyomaviral large T antigen or a functional equivalent thereof and B comprises a gene encoding the protein of interest under the functional control of the polyomaviral origin of replication or a functional equivalent thereof, wherein said cell lacks the capability to express a polyomaviral small T antigen or a functional equivalent thereof as well as the capability to express a polyomavirus capsid protein, the method further comprising the step of culturing said cell under conditions allowing expression of the recombinant protein of interest and harvesting the protein of interest from the cell culture.

Said gene encoding the protein of interest under the functional control of the polyomaviral origin of replication or a functional equivalent thereof may be provided on an episomal nucleotide such as a vector which may be introduced into said cell or in the alternative may be or may become part of the genome of said cell.

The expression "a gene encoding a protein of interest under the functional control of the polyomaviral origin of replication or a functional equivalent thereof" in this context means that the copy number of the gene encoding the protein of interest may be increased, for instance by amplification in the nucleus of the cell as a result of the interaction between a large T antigen and the origin of replication or a functional equivalent thereof leading to an increase in the expression of the protein of interest.

In that respect, the origin of replication may be any genetic element that is capable of initiating replication and/or amplification of the copy number of the gene encoding the protein of interest.

Genetic elements A and B may independently from each other be part of the genome of the cell, i.e. stably integrated into the genome. They may also be situated on an episomal polynucleotide independently from each other. It may also be envisaged that both elements A and B are on one and the same episomal polynucleotide.

A suitable genetic element for use in the above method comprises a DNA molecule that harbours the polyomaviral origin of replication, and encodes a functional polyomaviral large T antigen, and does not encode a functional polyomaviral small T antigen or functional equivalent thereof, and does not encode functional polyomaviral capsid proteins or functional equivalents thereof, and encodes the protein of interest.

In another embodiment, a suitable genetic element for use in the invention comprises a DNA molecule that harbours the polyomaviral origin of replication, and encodes the protein of interest. Said DNA molecule is capable of replication in a mammalian cell that provides the polyomavirus large T antigen in trans, i.e. the mammalian cell is capable of encoding the polyomavirus large T antigen or a functional equivalent thereof. A suitable example of such a cell is for instance the SuperVero cell. Such cell is permissive to the polyomavirus and may harbour such a DNA molecule not encoding functional polyomaviral small T antigen or functional equivalent thereof, and not encoding a functional polyomaviral capsid protein or functional equivalents thereof.

In the context of the present invention, the term "permissive to a polyomavirus" means capable of supporting the replication of polyomaviral DNA.

Unless the context clearly requires otherwise, throughout the description and the claims, the words 'comprise', 'comprising', and the like are to be construed in an inclusive sense as opposed to an exclusive or exhaustive sense; that is to say, in the sense of "including, but not limited to".

The expression "a polyomaviral large T antigen" or "functional equivalent thereof" in this context means a large T antigen obtainable from a polyomavirus or a fragment or analogue thereof that is capable of sustaining the multiplication of polyomaviral replicon DNA and of activating the polyomaviral late promoter in cells permissive for the polyomavirus.

The functionality of large T antigen or a fragment or analogue thereof can be tested by co-expressing an expression plasmid coding for the polyomavirus large T antigen or an equivalent thereof together with T antigen-deleted polyomaviral (early replacement) vector DNA in cells permissive to the wildtype polyomavirus and determining whether polyomavirus vector particles are produced. It may be concluded that polyomavirus large T antigen or a fragment or analogue thereof is a functional large T antigen if a single polyoma virus particle is produced in this assay. Such may be determined by electron microscopy or any other suitable method known in the art.

Such large T antigen coding domain on a DNA molecule of the invention may be devoid of the large intron of the polyomavirus early transcript harbouring the small T antigen-specific DNA sequences.

The expression "a polyomaviral small T antigen" or "functional equivalent thereof" in this context means a small T antigen obtainable from a polyomavirus or a fragment or analogue thereof that is capable of interacting with and/or inhibiting protein phosphatase 2A. The functionality of small T antigen can be tested using a binding assay between the polyomaviral small T antigen or an equivalent thereof and protein phosphatase 2A as described by Cho U.S., et al., PLoS Biology 5(8): e202, 2007. It may be concluded that the small T antigen or an equivalent thereof is a functional small T antigen when the interaction and/or inhibition in this assay is above background.

The expression "a polyomaviral capsid protein" or "functional equivalents thereof" in this context means capsid proteins (VP1, VP2 and/or VP3) obtainable from a polyomavirus or fragments or analogues thereof that are capable of packaging circular DNA molecules that harbor a polyomaviral origin of replication into polyomavirus(-like) particles.

In a preferred embodiment, the genetic element useful in the invention comprises a DNA molecule that encodes a selectable marker such as a marker selected from the group consisting of the neomycin resistance gene, puromycin resistance gene, hygromycin resistance gene and other antibiotic resistance markers.

It may be clear to the skilled addressee that the DNA molecules useful in the present invention may also include one or more other components commonly found in cloning and expression plasmids. Such components may include, but are not limited to, a multiple cloning site (a polylinker region) to allow easy sub-cloning of DNA restriction endonuclease fragments into other plasmids, an origin of replication to allow replication of the plasmid in Escherichia coli and the like (Sambrook et al., Molecular cloning, 2001).

The coding domains of the proteins of interest may be operably linked to suitable regulatory DNA regions for being transcribed and expressed in a mammalian cell. For transcription of a coding domain, regulatory DNA regions including a promoter, enhancer, splice donor and acceptor sites, or polyadenylation site may be used to transcribe the DNA of the coding domain in a mammalian cell.

By "promoter" is meant a sequence of nucleotides from which transcription may be initiated of DNA operably linked downstream (i.e. in the 3' direction on the sense strand of double stranded DNA).

"Operably linked" means joined as part of the same nucleic acid molecule, suitably positioned and oriented for transcription to be initiated from the promoter. DNA operably linked to a promoter is "under transcriptional initiation regulation" of the promoter. The promoter may be a constitutive promoter, an inducible promoter or tissue-specific promoter. The terms "constitutive", "inducible" and "tissue- specific" as applied to a promoter is well understood by those skilled in the art.

The promoter is preferably derived from viruses, including 5'-long terminal repeats from retroviruses and lentiviruses, the polyomavirus early and late promoters, the human cytomegalovirus immediate early promoter (CMVie), or from mammalian cells, including the human elongation factor 1 alpha promoter (EF-1 alpha) and the like.

By "polyadenylation signal" is meant a sequence of nucleotides from which transcription may be terminated and a poly-A tail is added to the transcript. As polyadenylation signal any polyadenylation signal applicable in human or animal cells can be used. Such promoters and polyadenylation signals are readily available and are well known in the art (vide WO 97/32016; US 5,593, 874; US 5, 698, 425, US 5,712, 135; US 5, 789, 214 and US 5, 804, 693).

In the context of the present invention, the term " protein of interest" includes any peptide or protein. Accordingly, the term includes, but is not limited to, insulin, alpha or beta interferon, hepatitis B surface antigen, GM-CSF, G-CSF, blood clotting factor VII VIII or IX, erythropoietin, streptokinase, human growth hormone, relaxin, rennin, interleukin, tumor necrosis factor, follicle stimulating factor and antibody or a functional equivalent thereof.

In a preferred embodiment the protein of interest is a therapeutic protein. In another embodiment the protein of interest is a monoclonal antibody. In a further embodiment, the protein of interest is suitable for use as a vaccine.

The DNA molecule useful in the invention may preferably be capable of episomal replication and long-term maintenance in the nucleus of a mammalian cell permissive to the cognate polyomavirus, allowing pseudo-stable expression of the recombinant protein(s) encoded by the genetic elements in said mammalian cell.

It may be clear to the skilled addressee that in the context of the present application, the term "pseudo-stable" refers to expression of a desired protein beyond 72 hours after introducing the DNA molecule(s) of the invention in the mammalian cell. Preferably, the replication and retention of the DNA molecule(s) of the invention expressing the recombinant protein of interest lasts for more than three weeks.

Preferably, DNA replication is initiated by interaction of the polyomaviral large T antigen or a functional equivalent thereof with the polyomaviral origin of replication or functional equivalent thereof.

Methods for introducing DNA molecules into mammalian cells are known to a person skilled in the art. The simplest approach is physical introduction of naked DNA using a gene gun or by electroporation. Chemical introduction of naked DNA into mammalian cells can be done using cationic lipids or polymers. The DNA can be packaged with lipids into liposomes for efficient introduction into mammalian cells. Alternatively, the DNA can be packaged with polyomaviral capsid proteins into polyomavirus (pseudo-) virus particles for efficient introduction into mammalian cells.

In one aspect, the invention provides a mammalian cell permissive to a polyomavirus that stably expresses the polyomaviral large T antigen or functional equivalent thereof, and is incapable of expressing a functional polyomaviral small T antigen or functional equivalent thereof, and is incapable of expressing a functional polyomaviral capsid protein or functional equivalents thereof wherein said cell harbours a genetic element comprising a DNA molecule that encodes a protein of interest under the operational control of a polyomaviral origin of replication or functional equivalent thereof.

A genetic element comprising a DNA molecule that harbours the polyomaviral origin of replication, and encodes the protein of interest may preferably be capable of replication and is not encapsidated into polyomavirus(-like particles) in said cell according to the invention.

A cell according to the invention may now be obtained by the skilled person using the information provided herein and using his ordinary skills. In particular, he may follow the guidance provided in the examples in order to arrive at a cell line comprising cells according to the invention.

Cell lines for use in a method according to the present invention may be derived from conventional mammalian cell lines permissive for a polyomavirus. Such cells may be used for the production of recombinant proteins since they are able to replicate circular DNA molecules harbouring the polyomavirus origin of replication in the presence of the polyomaviral large T antigen.

In a preferred embodiment of the invention, the cell is derived from a polyomavirus permissive cell line, such as a Vero cell line (African Green Monkey kidney cell line ECACC 88020401 European Collection of Cell Cultures, Salisbury, Wiltshire, UK).

In a further preferred embodiment of the invention the cell line is derived from a rodent cell line such as CHO-K1 (Chinese Hamster Ovary cell line ECACC European Collection of Cell Cultures, Salisbury, Wiltshire, UK).

In addition, a cell line for use in a method according to the invention may be derived from any suitable cell line known in the art such as MDCK, PER.C6, HEK293, HEK293T, CV1 and the like.

Suitable polyomaviral origins of replication may advantageously be selected from a polyomavirus selected from the group consisting of hamster polyomavirus, murine polyomavirus, monkey polyomavirus such as SV40 and human polyomavirus such as BK, JC, WU, KI and Merkel Cell polyomavirus.

Suitable large T antigens for use in the present invention may advantageously be selected from a polyomavirus selected from the group consisting of hamster polyomavirus, murine polyomavirus, monkey polyomavirus such as SV40 and human polyomavirus such as BK, JC, WU, KI and Merkel Cell polyomavirus.

The present invention is herein exemplified in the following examples which provide experimental evidence that the method according to the invention yields faster and better results in a mammalian expression system, and moreover produces large amounts of recombinant protein of interest.

The examples disclose the generation of a set of plasmids encoding a gene of interest, in this case, secreted alkaline phosphatase (SEAP). This gene was placed under the transcriptional control of the SV40 early promoter, located within the origin of replication. The characteristics of plasmids pAM068, pAM069 and pAM070 are disclosed in table 1.

**Table 1**

| **Vector** | **Large T antigen** | **Origin of replication** | **Small T antigen** | **Protein of interest** | **Capsid proteins** |
|---|---|---|---|---|---|
| pAM068 | + | + | - | **SEAP** | - |
| pAM069 | - | + | - | **SEAP** | - |
| pAM070 | - | - | - | **SEAP** | - |
| Prior art | + | + | - | **ND** | + |

These plasmids were introduced into SuperVero cells and into Vero SF cells. SuperVero cells are capable of encoding the SV40 large T antigen in trans whereas the Vero SF cell is incapable of expressing a large T antigen.

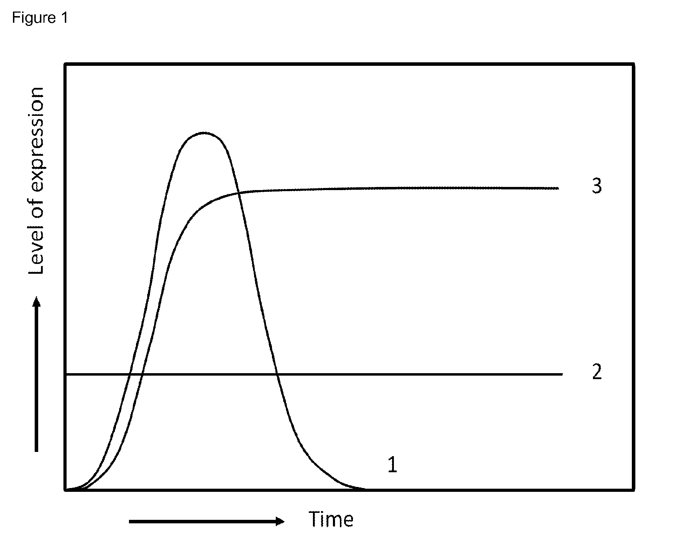

It was found that SuperVero cells transfected with DNA from the SV40 replicons pAM068 and pAM069 and Vero SF cells transfected with DNA from replicon pAM068 produced significantly more SEAP for a significantly longer period of time (Line 3 in Figure 1) compared to control Vero SF cells transfected with DNA from pAM069 and pAM070 and SuperVero cells transfected with DNA from pAM070 (Line 1 in Figure 1). A typical classical stable SEAP-producing cell line is represented with line 2 in Figure 1.

These results are also shown in Table 2.

**Table 2**

| Vector | Expression profile in Vero SF cells | Expression profile in SuperVero cells |
|---|---|---|
| pAM068 | **3** | **3** |
| pAM069 | **1** | **3** |
| pAM070 | **1** | **1** |
| Prior art | **1** | **1** |
| Constitutive producer cell line | **2** | **2** |

### Legend to the figures

Figure 1: Schematic representation of expression levels of a protein of interest in 3 different expression systems: 1 represents an expression profile that may be obtained with a polyomavirus expression system according to the prior art. The expression levels decrease rapidly after reaching a peak value because cells are destroyed by the production of viral particles. 2 represents the expression levels obtainable with an expression system employing a constitutive promoter according to the prior art. Line 3 represents the expression levels obtainable by a method according to the present invention.

### Examples

### Example 1: Construction of an expression plasmid encoding the SV40 large T antigen

A synthetic multiple cloning site (MCS) was designed containing restriction sites for Notl, Pacl, Sbfl, Pmel, Ascl and Clal. Two oligonucleotides were designed WdV436: 5'-GCCGCTTTATTAATTAAGCCCTGCAGGTTGTTTAAACTTGGCGCGCCTTAT-3'(SEQ ID NO: 1) and WdV437: 5'-CGAAATAATTAATTCGGGACGTCCAACAAATTTGAACCGCGCGGAATAGC -3'. (SEQ ID NO 2). Both oligonucleotides WdV436 and WdV437 were annealed to each other and ligated into pBluescript SK- (Promega), yielding the recombinant plasmid pAM007.

Two oligonucleotides were designed to introduce an additional Notl restriction site WdV452: 5'- CGGCGGCCGCGTAC -3' (SEQ ID NO: 3) and WdV453: 5'-GCGGCCGC -3'. Both oligonucleotides were annealed and ligated into pAM007, yielding the recombinant vector pAM008.

The expression vector pLenti6.3/V5DEST_verA (Invitrogen) was used as a template for cloning of the cytomegalovirus immediate early (CMVie) promoter using PCR. Two oligonucleotides were designed WdV286: 5'-TTGGCGCGCCTCAATATTGGCCATTAGCCATATTATTCATTGG-3' (SEQ ID NO: 4) and WdV220: 5'- GCTAGGTCGGAGGCGCCGGCCCTTGCCACGTAACCTTCGAACAG -3' (SEQ ID NO: 5) flanking the CMV promoter. Oligonucleotides WdV286 and WdV220 contained restriction sites AscI and Hindlll respectively. Subsequently, purified pLenti6.3/V5DEST_verA was subjected to PCR using oligonuleotides WdV286 and WdV220, yielding a CMV promoter DNA fragment. This fragment was AscI and HindIII digested and ligated into pBluescript SK-, yielding pAM009.

The expression vector pGL4.22 (Promega) was used as a template for cloning of the puromycin N-acetyltransferase antibiotic resistance gene using PCR. Two oligonucleotides were designed WdV454: 5'-CCACCCAAGCTTATGACCGAGTACAAGCCCACGGTGCG-3' (SEQ ID NO: 6) and WdV455: 5'- CGTACTGGGCGTTCGGGCCACGGACTGAGCTCGCCTAT -3' (SEQ ID NO: 7) flanking the puromycin N-acetyltransferase antibiotic resistance gene and containing restriction sites Hindlll and Xhol, respectively. Plasmid pGL4.22 was subjected to PCR using oligonucleotides WdV454 and WdV455, yielding the puromycin N-acetyltransferase cDNA. This fragment was Hindlll and Xhol digested and ligated into pAM009, yielding pAM010.

The expression vector pEF5/FRT/5-DEST (Invitrogen) was used as a template for cloning of the BGH polyadenylation signal using PCR. Two oligonucleotides were designed WdV456: 5'- CAACCGCTCGAGCTGTGCCTTCTAGTTGCCAGCCATC-3' (SEQ ID NO: 8) and WdV457: 5'- CGGGGTACCCCATAGAGCCCACCGCATCCCC -3' (SEQ ID NO: 9) flanking the polyadenylation signal and containing restriction sites Xhol and Kpnl respectively. Plasmid pEF5/FRTN5-DEST was subjected to PCR using oligonucleotides WdV456 and WdV457, yielding the BGH polyadenylation signal cDNA. This fragment was Xhol and Kpnl digested and ligated into pAM010, yielding pAM011.

Plasmids pAM008 was digested with Ascl and Pmel and the DNA fragment comprising the puromycin N-acetyltransferase coding domain was purified from an agarose gel and ligated into pAM008, yielding pAM012.

DNA of a full-length SV40 DNA clone (ATCC number VRMC-2) was used as template for cloning of the SV40 T antigen coding region using PCR. Two oligonucleotides were designed WdV408: 5'-ACCATGGATAAAGTTTTAAACAGAGAGGAATCTTTGCAGC -3' (SEQ ID NO: 10) containing an attB1 recombination site and WdV409: 5'-TTATGTTTCAGGTTCAGGGGGAGGTGTGGGAGG -3' (SEQ ID NO: 11) containing an attB2 recombination site. WdV408 and WdV409 were used to PCR amplify the genomic T antigen coding region. Subsequently, a gateway entry clone was generated from the generated DNA fragment and pDONR221, resulting in pAM013. A T antigen expression plasmid was generated by gateway recombination between pAM013 and pEF5/FRT/V5-DEST, resulting in pAM014.

The Notl and Pmel restriction sites in plasmid pAM014 were eliminated by Notl and Pmel digestion of pAM014 followed by a T4 DNA polymerase treatment and re-ligation, yielding pAM015. The T antigen expression cassette was subsequently isolated by a Sphl digestion followed by a T4 DNA polymerase treatment and a Nrul digestion.

In order to generate a shuttle plasmid two oligonucleotides were designed WdV448: 5'-TCCTGCAGGCGGGGTACCCTAGTCTAGACTAGCCGCGGGGAGTTTAAACAGCT - 3'(SEQ ID NO: 12) and WdV449: 5'-GTTTAAACTCCCCGCGGCTAGTCTAGACTAGGGTACCCCGCCTGCAGGAGTAC -3' (SEQ ID NO: 13).

Oligonucleotides WdV448 and WdV449 were annealed generating a DNA fragment that contains the Kpnl, Sbfl, Kpnl, Xbal, Sacll, Pmel and Sacl restriction sites. This DNA fragment was ligated into Kpnl and Sacl digested pBluescript SK-(Promega), yielding pAM016. Plasmid pBluescript SK- was digested with Kpnl and Xbal and the MCS DNA fragment was isolated from an agarose gel. The MCS DNA fragment was ligated into pAM016 digested with Kpnl and Xbal, resulting in pAM017.

The EF1 alpha driven T antigen expression cassette from pAM015 was isolated by a Nrul and Sphl digest followed by a T4 DNA polymerase treatment. The resulting DNA fragment was cloned into pAM017 digested with EcoRV, resulting in pAM018.

Plasmid pAM018 was digested with Sbfl and Pmel and the DNA fragment comprising the T antigen expression cassette was isolated from an agarose gel and cloned into pAM012 digested with Sbfl and Pmel, resulting in pAM019.

Four oligonucleotides were designed WdV487: 5'-GCAGGCTACCATGGATAAAGTTTTAAACAGAGAG-3' (SEQ ID NO: 14) and WdV490: 5'- GAAACCTCCGAAGACCCTACGTTGACTCTAAGGTTGGATACCTTGACTACTTACC - 3' (SEQ ID NO: 15) WdV:489 5' CTTTGGAGGCTTCTGGGATGCAACTGAGATTCCAACCTATGGAACTGATGAATGGG-3' (SEQ ID NO: 16) and WdV488: 5'- AGGAATGTTGTACACCATGCATTTTAAAAAGTC - 3' (SEQ ID NO: 17).

Oligonuleotides WdV487 and WdV490 and oligonucleotides WdV489 and WdV488 were used to amplify the first and the second exon of the SV40 large T antigen respectively. Both generated DNA fragments were subsequently subjected to a fusion PCR using oligonucleotides WdV487 and WdV488.

The generated DNA fragment comprising the SV40 large T antigen coding region was digested with Ncol and Nsil and cloned into likewise digested pAM019, resulting in pAM001.

In summary, pAM001 contains an EF1 alpha promoter upstream of the large T antigen coding region and a CMVie promoter upstream of the puromycin N-acetyltransferase coding region.

### Example 2: Generation of a Vero producer cell line.

Vero cells (Sigma-Aldrich order number: 88020401) were propagated and adapted to serum free culture DMEM medium (Invitrogen, product code: 41966-052). Adaptation to serum free conditions was performed by gradually reducing fetal bovine serum from 8, 6, 4, 2 and 0% in the medium each passage. From then the Vero-Serum Free (Vero-SF) cells were cultured in OptiPro SFM medium (Invitrogen) containing 2% L-glutamine at 37°C and 5% CO2.

Vero-SF cells were transfected with pAM001 DNA using the transfection agent Exgen 500 (Fermentas, product code: R0511) according to the supplier's prescriptions. The transfected Vero-SF cells were subsequently selected for integration of the SV40 large T expression gene cassette into the chromosomal DNA by adding 2 µg/ml puromycine to the cell culture medium. Surviving colonies were isolated and propagated in OptiPro SFM medium containing 2 µg/ml puromycine and 2% L-glutamine. Puromycin-resistant cells were transferred OptiPro SFM medium containing 2% L-glutamine and 10% DMSO and stored at -156°C.

One puromycin-resistant Vero clone denoted Vero-SF001-86 expressed the SV40 large T antigen was selected for further experiments. A cell subclone of Vero-SF001-86 denoted Vero-SF001-86-01 or SuperVero was generated by limited dilution that stably expresses SV40 large T antigen.

### Example 3: Construction of SV40-based replicon plasmids

Six oligonucleotides were designed: WdV101: 5'-CCGCTCGAGTTGCGGCCGCTGTGCCTTCTAGTTGCCAGCCATC -3' (SEQ ID NO: 18, containing a Xhol and a Notl restriction site) and WdV102: 5'-GGTACCATAGAGCCCACCGCATCCCCAGCATGCC -3' (SEQ ID No.19) (containing a Kpnl restriction site) and WdV103: 5'-GGCCGCTTTATTAATTAAGCCCTGCAGGTTGTTTAAACTTGGCGC GCCTTAT-3'(SEQ ID NO: 20, containing from 5' to 3' subsequently a Notl sticky restriction site, a Padl, Sbfl, Pmel and an AscI intact restriction site and a Clal sticky restriction site) and WdV104: 5'-CGATAAGGCGCGCCAAGTTTAAACAACCTGCAGGGCTTAATTAAT AAAGC -3' (SEQ ID No. 21) (contains from 3' to 5' subsequently a NotI sticky restriction site, a PadI, SbfI, Pmel and an Ascl intact restriction site and a Clal sticky restriction site) and WdV105: 5'-CGGGATCCAGACATGATAAGATACATTG -3' (SEQ ID NO: 22, containing a BamHI restriction site) and WdV106: 5'-ATAGTTTAGCGGCCGCAACTTGTTTATTGCAGCTTATAATGG -3' (SEQ ID NO: 23, containing a Notl restriction site).

Purified plasmid DNA of the SV40 vector pSL-PL (De La Luna S., et al., Journal of General Virology 74: 535-539, 1993) was subjected to PCR using oligonucleotides WdV105 and WdV106. The resulting amplified DNA fragment comprises the SV40-polyadenylation signal flanked by a BamHl restriction site at the 5'-end and a Notl restriction site at the 3'-end. This SV40 polyadenylation signal fragment was digested with BamHl and Notl and the resulting 150 bp long DNA fragment was isolated from an agarose gel and cloned into a likewise digested pBluescript SKM plasmid (Promega), yielding pAM002.

Purified pEF5/FRT/V5-Dest (Invitrogen) plasmid DNA was subjected to PCR using oligonucleotides WdV101 and WdV102. The resulting amplified DNA fragment comprising the bovine growth hormone (BGH) polyadenylation signal flanked by subsequently a Xhol and a Notl restriction site at the 5' end and a Kpnl restriction site at the 3' end. This BGH polyadenylation signal fragment was digested with Kpnl and Notl, and the resulting 250 bp long DNA fragment was isolated from an agarose gel and ligated into the likewise digested pAM002 plasmid. Transformation with this ligation mixture was performed in a methylation insensitive E. coli strain. This resulted in plasmid pAM003.

The two complementary oligonucleotides WdV103 and WdV104 were annealed by incubating them in a water bath that was cooling down autonomously from boiling temperature to room temperature, yielding a DNA linker containing subsequently a Notl sticky restriction site, a Pacl, Sbfl, Pmel and an Ascl intact restriction site and a Clal sticky restriction site. This linker was ligated into the pAM003 plasmid that was digested with Notl and Clal and isolated from an agarose gel. The ligation mixture was subsequently used to transform a methylation insensitive E. coli strain, yielding pAM004.

Purified plasmid DNA of the SV40 vector pSL-PL was digested with Clal and BamHI. The resulting 2.6 kb DNA fragment that contains the SV40 origin and the SV40 late region is purified from agarose and cloned into likewise digested pAM004. This resulted in the new SV40 vector plasmid pAM005.

DNA of a full-length SV40 DNA clone (ATCC number VRMC-2) was used as template for cloning of the SV40 Large T antigen coding region using PCR. In order to replace the wild-type T antigen coding region by the large T antigen region, four oligonucleotides were designed: WdV051: 5'-TCTAGGCGCGCCGATGGATAAAGTTTTAAACAGAG-3' (SEQ ID NO: 24), WdV490: 5'-GAAACCTCCGAAGACCCTACGTTGACTCTAAGGTTGGATACCTTGACTACTTACC -3' , (SEQ ID NO: 25), WdV:489 5'-CTTTGGAGGCTTCTGGGATGCAACTGAGATTCCAACCTATGGAACTGATGAATGGG-3' (SEQ ID NO: 26), and WdV052: 5'- TCCTTAATTAATTATGTTTCAGGTTCAGG -3' (SEQ ID NO: 27),

Oligonuleotides WdV051 and WdV490 and oligonuleotides WdV489 and WdV052 were used to amplify the first and the second exon of the SV40 large T antigen respectively. Both generated DNA fragments were subsequently subjected to a fusion PCR using oligonuleotides WdV051 and WdV052.

The generated DNA fragment comprising the SV40 large T antigen coding region was digested with Ascl and Pacl and cloned into likewise digested pAM005, resulting in pAM064.

The two complementary oligonuleotides WdV053 5'-GCAGTACTGGTTTAAACCAGATCTGGCGCCCCTGCAGGGGATCCTA-3' (SEQ ID NO: 28), and WdV054 5'-TAGGATCCCCTGCAGGGGCGCCAGATCTGGTTTAAACCAGTACTGC -3' (SEQ ID NO: 29), were annealed by incubating them in a water bath that was cooling down autonomously from boiling temperature to room temperature, yielding a DNA linker containing subsequently a Scal blund restriction site, a Pmel, Bglll, Narl, Sbfl, and a BamHl restriction site.

The late region (encoding the SV40 capsid proteins agno, VP1, VP2 and VP3) of pAM064 was removed by a partial Ncol digest at the agno protein's start codon. The 3' overhang of the Ncol site was removed by a DNA polymerase I Klenow reaction.

Secondly, the fragment was purified and digested with BamHl. Subsequently, the DNA linker containing a Scal blund restriction site, a Pmel, Bglll, Narl, Sbfl, and a BamHl restriction was digested with Scal and BamHl and both DNA fragments were, yielding pAM065. Two oligonuleotides were designed WdV001 5'-AGCTTTAGTTTAAACACAAGTTTGTACAAAAAAGCTGAACG-3' (SEQ ID NO: 30), and WdV002 5'- AGATACCCTGCAGGACCACTTTGTACAAGAAAGC-3' (SEQ ID NO: 31), containing respectively a Pmel and Sfbl restriction site. The pEF5/FRT/N5-Dest was used as template to isolated the single gateway cassette by PCR amplification using primers WdV055 and WdV056. Subsequently, the purified PCR fragment was digested, gel purified and ligated into the likewise digested pAM065 and propagated in ccdB survival cells (Invitrogen). This resulted the SV40 late replacement vector pAM066. Two oligonuleotides were designed WdV056 5'-GGGACAAGTTTGTACAAAAAAGCAGGCTTAATGCTGCTGCTGCTGCTG -3' (SEQ ID NO: 32), and WdV057 5'-GGGGACCACTTTGTACAAGAAAGCTGGGTATCATGTCTGCTCGAAGCG -3' (SEQ ID NO: 33), containing respectively an AttB1 and AttB2 recombination site. WdV056 and WdV057 were used to PCR amplify the SEAP *(secreted* alkaline phosphatase) protein coding sequence from pSEAP2-basic plasmid (Clontech).

The PCR fragment was gel purified and subject to a BP recombination reaction with pDONR221 (Invitrogen), resulting in the SEAP entry clone pAM067. Subsequently, an LR recombination reaction was performed with DNA constructs pAM067 and pAM066, resulting in an SV40-based late replacement replicon encoding SEAP pAM068.

An LR recombination reaction was performed with DNA constructs pAM067 and purified pcDNA6.2/V5-DEST (Invitrogen) plasmid DNA, resulting in an SV40-based early plus late replacement replicon encoding SEAP pAM069.

An EF1 alpha-driven SEAP expression plasmid was constructed and used as a control expression vector. A gateway LR recombination reaction was performed with DNA constructs pAM067 and pEF5/FRT/V5-Dest. This resulted in an EF1-alpha driven SEAP expression vector pAM070.

### Example 4: production of SEAP recombinant protein in Vero cells

SuperVero and control Vero SF cells were seeded 120.000 cells per well and subsequently transfected with purified replicon DNA encoding SEAP pAM068, pAM069 or pAM070. At several time points after transfection supernant was collected, concentrated and SEAP *(secreted* alkaline phosphatase) expression was measured using the GreatEscApe SEAP chemiluminescence detection kit (Clontech) according the manufacturer's recommendations. SuperVero cells transfected with DNA from the SEAP SV40 replicons pAM068 and pAM069 and Vero SF cells transfected with DNA from replicon pAM068 produced significantly more SEAP for a significantly longer period of time (Line 3 in Figure 1) compared to control Vero SF cells transfected with DNA from pAM069 and pAM070 and SuperVero cells transfected with DNA from pAM070 (Line 1 in Figure 1). A typical classical stable SEAP-producing cell line is represented with line 2 in Figure 1.

## Claims

1. Method for the production of a protein of interest in a mammalian cell permissive to a polyomavirus comprising the genetic elements A and B wherein element A encodes a polyomaviral large T antigen or a functional equivalent thereof and B comprises a gene encoding a protein of interest under the functional control of the polyomaviral origin of replication or a functional equivalent thereof, wherein said cell lacks the capability to express a polyomaviral small T antigen or a functional equivalent thereof as well as the capability to express a polyomavirus capsid protein, the method comprising the step of culturing said cell under conditions allowing expression of the protein of interest and harvesting the protein of interest from the cell culture.

2. Method according to claim 1 wherein the genetic element B encoding the gene of interest is situated on an episomal polynucleotide.

3. Method according to claims 1 or 2 wherein the genetic element A is situated on an episomal polynucleotide.

4. Method according to claim 1 wherein the genetic elements A and/or B are stably integrated into the genome of said cell.

5. Method according to claims 1 - 4 wherein said cell is a SuperVero cell or a Vero cell.

6. A mammalian cell permissive to a polyomavirus comprising the genetic elements A and B wherein A encodes a polyomaviral large T antigen or a functional equivalent thereof and B comprises a gene encoding a protein of interest under the functional control of the polyomaviral origin of replication or a functional equivalent thereof, wherein said cell lacks the capability to express a polyomaviral small T antigen or a functional equivalent thereof as well as the capability to express a polyomavirus capsid protein.
